# EUROPEAN PATENT SPECIFICATION

(11) **EP 3 854 386 B1**
(45) Date of publication and mention of the grant of the patent: **22.01.2025**
(21) Application number: 20212959.9
(22) Date of filing: 10.12.2020
(51) Int. Cl.: A61K 9/20, A61K 31/395

(54) **AN ORALLY DISINTEGRATING PHARMACEUTICAL COMPOSITION COMPRISING NEFOPAM AND PROCESS FOR PREPARING THE SAME**
ORAL ZERFALLENDE PHARMAZEUTISCHE ZUSAMMENSETZUNG MIT NEFOPAM UND VERFAHREN ZUR HERSTELLUNG DAVON
COMPOSITION PHARMACEUTIQUE À DÉSINTÉGRATION ORALE COMPRENANT DU NÉFOPAM ET SON PROCÉDÉ DE PRÉPARATION

(30) Priority: 21.01.2020 IN 202011002685
(43) Date of publication of application: 28.07.2021
(73) Proprietor: Athena Pharmaceutiques SAS, 78430 Louveciennes (FR)
(72) Inventor: Chaudhari, Mahendra, 400602 Thane (West) Maharashtra (IN)
(74) Representative: Dr. Solf & Zapf Patent- und Rechtsanwalts PartG mbB

(56) References cited:
- CN-A- 1 569 004
- CN-A- 110 279 691
- CN-K1- 1 569 004
- US-A- 6 106 861
- US-A1- 2006 105 039
- US-A1- 2020 323 878

## Description

### FIELD OF THE INVENTION

The present invention relates to an orally disintegrating pharmaceutical composition comprising Nefopam and pharmaceutically acceptable inert excipients and a process for preparing the same.

### BACKGROUND OF THE INVENTION

Nefopam is a centrally acting non-narcotic analgesic primarily used to treat moderate to severe, acute or chronic pain. Chemically it is 5-methyl-1-phenyl-3, 4, 5, 6-tetrahydro-1H-2, 5-benzoxazocine hydrochloride with following chemical structure.

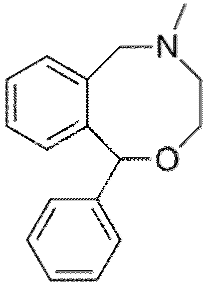

It is available commercially in UK as a film coated tablet available in 30mg strength. It is approved for the relief of acute and chronic pain, including post-operative pain, dental pain, musculo-skeletal pain, acute traumatic pain and cancer pain. The commercially available product tablet core constituents are: Calcium hydrogen phosphate dehydrate, Microcrystalline cellulose, Starch, pregelatinised, Magnesium stearate, Hydrogenated vegetable oil, Silica, colloidal anhydrous. The Film-coat constituents are Hypromellose (E464), Titanium dioxide (E171), Lactose monohydrate, Macrogol 3000, Triacetin.

US application US20110275626 discloses a formulation for oral transmucosal administration of at least one active principle for treating a spastic crisis, including: at least one active principle present in base form and/or in salt form, chosen from peripheral action antispasmodics and nefopam; an aqueous alcohol solution titrating at least 35° alcohol; and optionally, another active principle present in base form and/or in salt form, chosen from central action analgesics; the active principle(s) being present in a state of stable and complete dissolution in the aqueous alcohol solution.

CN 110 279 691 A discloses a pharmaceutical composition, namely a granule, having multiple active ingredients. The composite comprises 25 parts of Nefopam, 75 parts of indomethacin, 50 parts of 5-caffeoylquinic acid, 50 parts of lactose, 80 parts of microcrystalline cellulose, 15 parts of povidone XL, 6 parts of sodium carboxymethyl starch.

CN 1 569 004 A provides a dripping pill. Dripping pills are small globules derived from a dripping pill preparation process, which involves melt dispersion, dripping and consolidation. Said dripping pill preparation process according to CN 1 569 004 A involves a Nefopam powder to be mixed into a molten matrix and condensing the mixture into pills in a coolant. However, dripping pills and tablets are different products and steps of producing a dripping pill and steps of producing a tablet are not compatible.

US 2006/105039 A1 discloses an orally disintegrating tablet and a process for producing such an orally disintegrating tablet by mixing the excipients with the active ingredient to form core particles. Further, US 2006/105039 A1 does not disclose any usability for analgesics and Nefopam.

US 6 106 861 A discloses a tablet having a systematically pleasant texture. Therefore, a tablet is providing having an active ingredient including a coating for the pleasant texture. However, the content of the active ingredient per se cannot be derived. Further, US 6 106 861 A is silent regarding Nefopam and only generally teaches to use analgesic microcrystals.

Orally disintegrating tablets (ODTs) differ from conventional tablets in that they are designed to be dissolved/disintegrated in an oral cavity rather than swallowed whole. Accordingly, ODTs have been developed such that the same medicine can be orally administered in a simple and effective manner to children, adults and people with impaired swallowing function and similar conditions. Even a patient who is not suffering from any swallowing limitation will find it much easier to take an ODT as compared to a conventional tablet. An additional reason to use an orally disintegrating tablet is the convenience of a tablet that can be taken without water. In view of ease of administration, the ODTs have a rapid disintegration rate as compared to conventional tablets.

H Y Guan; Preparation of Nefopam Hydrochloride Orally Disintegrating Tablet, a Chinese thesis discloses preparation of the tablets formulated according to the orthogonal design by direct compression. β-cyclodextrin inclusion complex techniques was evaluated to mask the bitter taste of nefopam hydrochloride. The stability was investigated through accelerated test. The in vivo behavior of the tablets was evaluated in beagle dogs after administration of orally disintegrating tablets and ordinary tablets in the market by a randomized, crossover and self-control trial. Pharmacokinetic parameters of the formulations were compared, and the relative bioavailability and bioequivalence were calculated.

There is a need of orally disintegrating tablet composition comprising Nefopam hydrochloride to provide quick relief of the acute pain. Accordingly, the inventors of the present invention have developed orally disintegrating pharmaceutical composition comprising Nefopam and pharmaceutically acceptable inert excipients.

### SUMMARY OF THE INVENTION

To achieve the foregoing and other objects and needs, the present invention provides an orally disintegrating pharmaceutical composition comprising Nefopam and pharmaceutically acceptable inert excipients according to claim 1 and a process for preparing the same according to claim 7.

In first aspect, the present invention provides an orally disintegrating pharmaceutical composition comprising Nefopam and pharmaceutically acceptable inert excipients, wherein the composition comprises 5 to 10 % w/w of Nefopam with respect to the total weight of the composition.

Further, the present invention provides an orally disintegrating tablet comprising:
a. Nefopam in the form of taste masked pellets or granules;
b. about 1 % to about 50 % w/w Diluent;
c. about 5% to about 50 % w/w Disintegrant;
d. about 0.05 % to about 2 % w/w of Glidant; and
e. about 0.05 % to about 2 % w/w of Lubricant.

wherein the % are with respect to the total weight of the tablet
wherein the disintegration time of the tablet is less than 60 seconds as measured by the in vitro disintegration test described in US Pharmacopoeia 701, without disks.

In a further aspect, the present invention provides a process for preparing an orally disintegrating pharmaceutical tablet comprising Nefopam according to the first aspect, the process comprising the steps of:
(a) Preparing pellets or granules of Nefopam;
(b) Taste masking the pellets or granules;
(c) Preparing a blend comprising various pharmaceutically acceptable inert excipients;
(d) Mixing the blend with Nefopam pellets or ganules and lubricating it;
(e) Compressing the lubricated blend to form a tablet.

### DETAILED DESCRIPTION OF THE INVENTION

The exemplary embodiments described herein detail for illustrative purposes are subject to many variations in structure and design. It should be emphasized, however, that the present invention is set out in the appended set of claims. Also, it is to be understood that the phraseology and terminology used herein is for the purpose of description and should not be regarded as limiting.

The use of terms "including," "comprising," or "having" and variations thereof herein is meant to encompass the items listed thereafter and equivalents thereof as well as additional items.

Further, the terms, "a" and "an" herein do not denote a limitation of quantity, but rather denote the presence of at least one of the referenced item.

As used herein, "about" will be understood by persons of ordinary skill in the art and will vary to some extent depending upon the context in which it is used. If there are uses of the term in which are not clear to persons of ordinary skill in the art, given the context in which it is used, "about" will mean up to plus or minus 10% of the particular term.

As used herein, "treatment" or "treating" are used interchangeably. These terms refer to an approach for obtaining beneficial or desired results including but not limited to a therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder. For prophylactic benefit, the compositions may be administered to a patient at risk of developing a particular disease, or to a patient reporting one or more of the physiological symptoms of a disease, even though a diagnosis of this disease may not have been made. Treatment includes preventing the disease, that is, causing the clinical symptoms of the disease not to develop by administration of a protective composition prior to the induction of the disease; suppressing the disease, that is, causing the clinical symptoms of the disease not to develop by administration of a protective composition after the inductive event but prior to the clinical appearance or reappearance of the disease; inhibiting the disease, that is, arresting the development of clinical symptoms by administration of a protective composition after their initial appearance; preventing reoccurring of the disease and/or relieving the disease, that is, causing the regression of clinical symptoms by administration of a protective composition after their initial appearance.

In first aspect, the present invention provides an orally disintegrating pharmaceutical composition comprising Nefopam and pharmaceutically acceptable inert excipients, wherein the composition comprises 5 to 10 % w/w of Nefopam with respect to the total weight of the composition. Specifically, the composition comprises 5% or 5.5% or 6% or 6.5% or 7% or 7.5% or 8% or 8.1% or 8.2% or 8.3% or 8.4% or 8.5% or 8.6% or 8.7% or 8.8% or 8.9% or 9% or 9.5% or 10% w/w of Nefopam with respect to the total weight of the composition.

The term Nefopam as used herein includes all its salts, esters, enantiomers and isomers. Specifically, Nefopam is in the form of Nefopam Hydrochloride salt.

Nefopam as used herein is in the form of pellets or granules. Pellets are spherical or nearly spherical, free flowing granules with a narrow size distribution. The pellets are generally produced via a pelletization process whereby a powder blend consisting of drug and excipient particles is agglomerated into spherical granules. Pellets provide with a high degree of flexibility during the design and development of oral dosage forms. They can be divided into desired dose strengths without formulation or process changes, and also be blended to deliver incompatible bioactive agents simultaneously or particles with different release profiles at the same site or at different sites within gastrointestinal tract. Pellets provide development of formulation with high degree of flexibility due to free flowing characteristic. Various pelletization techniques include agitation, compaction, compression, extrusion-spheronization, powder or solution layering, spray drying and spray congealing and the like. The granules may be formed using the techniques like slugging or compaction or aqueous or non-aqueous wet granulation.

Specifically, the present invention uses the layering technique to prepare the Nefopam pellets or granules. It includes deposition of successive layers of Nefopam from solution, suspension or dry powder on crystals or granules of the same material or inert starter seeds. The diameter of non pareil seeds is in the range of 250 - 355µm. The fine particle size of the non pareil seeds doesn't cause any grittiness in tongue after the disintegration of the tablet.

Non-Pareil Seeds are spherical particles of uniform diameter that are practically inert, tasteless and odorless. Non-Pareil seeds are primarily composed of inert substances like starch, lactose. The non-Pareil seeds work as a base upon which drugs are coated. The Non-Pareil seeds are given a protective barrier.

According to an embodiment, the pellets or granules of Nefopam are taste masked.

The taste-masking techniques are applied to mask or overcome the bitter or unpleasant taste of active pharmaceutical ingredients/drugs to achieve patient acceptability and compliance. The commonly used industrial techniques/methods of taste-masking include organoleptic methods, polymer coating, hot-melt extrusion, microencapsulation, complexation, and spray-drying.

The taste of masking of Nefopam pellets or granules is done using aqueous dispersion of poly (meth) acrylate polymer. The commercially available grades of poly (meth) acrylate polymer like Eudragit NE 30D or Eudragit E by Evonik may be used for the purpose of taste masking. These taste masking agents are insoluble in saliva and prevent Nefopam to be in contact with saliva.

By the term "pharmaceutically acceptable inert excipients", it is meant any of the components of a pharmaceutical composition other than active ingredients and which are approved by regulatory authorities or are generally regarded as safe for human or animal use. The pharmaceutically acceptable inert excipients are used during the palletization or taste masking or coating or tableting compression.

Examples of pharmaceutically acceptable inert excipients include, but are not limited to diluents, binders, sweetening agent, flavorants, disintegrants, solvents, lubricant and glidants. A combination of excipients may also be used. The amount of excipient(s) employed will depend upon how much active agent is to be used. One excipient can perform more than one function as well. The commercially available grades of the excipients can also be used for the purpose of using in the composition according to the invention.

Diluents include, but are not limited to confectioner's sugar, compressible sugar, dextrates, dextrin, dextrose, fructose, lactitol, mannitol, sucrose, starch, lactose, xylitol, sorbitol, talc, microcrystalline cellulose, calcium carbonate, calcium phosphate dibasic or tribasic, calcium sulphate and other materials known to one ordinarily skilled in the art and mixtures thereof.

Binders include, but are not limited to, starches such as potato starch, wheat starch, corn starch (maize starch); microcrystalline celluloses; celluloses such as hydroxypropyl cellulose, hydroxyethyl cellulose, hydroxypropyl methylcellulose (HPMC), ethyl cellulose, sodium carboxymethylcellulose; natural gums like acacia, alginic acid, guar gum; liquid glucose, dextrin, povidone, syrup, polyethylene oxide, polyvinylpyrrolidone, poly-N-vinyl amide, polyethylene glycol, gelatin, poly propylene glycol, tragacanth and other materials known to one ordinarily skilled in the art and mixtures thereof.

The sweetening agents (also known as sweeteners) include, but are not limited to, aspartame, saccharin sodium, dipotassium glycyrrhizinate, stevia, thaumatin, acesulfame K, sucralose, and other materials known to one ordinarily skilled in the art and mixtures thereof.

The flavorants include natural or synthetic flavorants. The natural flavorant may be an essential oil, oleoresin, essence or extractive. The flavoring constituents may also be derived from a spice, fruit or fruit juice, vegetable or vegetable juice, whose significant function in the product is for flavoring purpose.

The disintegrants include, but are not limited to, starch, croscarmellose sodium, polyvinyl pyrrolidone, sodium starch glycolate, microcrystalline cellulose and other materials known to one ordinarily skilled in the art and mixtures thereof.

Solvents include, but are not limited to purified water, acetone, ethyl alcohol, isopropyl alcohol dichloromethane and other materials known to one ordinarily skilled in the art and mixtures thereof.

Lubricants may be selected from, but are not limited to, those conventionally known in the art such as Mg, Al or Ca or Zn stearate, polyethylene glycol, glyceryl behenate, mineral oil, sodium stearyl fumarate, stearic acid, hydrogenated vegetable oil, talc and other materials known to one ordinarily skilled in the art and mixtures thereof.

Glidants include, but are not limited to, silicon dioxide; magnesium trisilicate, powdered cellulose, starch, talc and tribasic calcium phosphate, calcium silicate, magnesium silicate, colloidal silicon dioxide, silicon hydrogel and other materials known to one ordinarily skilled in the art and mixtures thereof. Preferably, the glidant is colloidal silicon dioxide.

The pharmaceutical composition according to the present invention is in the form of a solid dosage form.

The present invention further provides an orally disintegrating tablet comprising:
a. Nefopam in the form of taste masked pellets or granules;
b. about 1 % to about 50 % w/w Diluent;
c. about 5% to about 50 % w/w Disintegrant;
d. about 0.05 % to about 2 % w/w of Glidant; and
e. about 0.05 % to about 2 % w/w of Lubricant
wherein the % are with respect to the total weight of the tablet.

According to the invention, the disintegration time of the tablet is less than 180 seconds, namely less than 60 seconds.

"Orally disintegrating tablet" means that the tablet disintegrates or disperses within 180 seconds as measured by the in vitro disintegration test described in US Pharmacopoeia 701, without disks. Such a disintegration test result is reasonably related to the actual disintegration time experienced by a mammal when placed in the oral cavity. The disintegration of the tablet means that the tablet shape/form is destroyed but does not necessarily mean that the entire tablet is dissolved. If coated particles of the active agent are contained within the tablet, as described hereinafter, such particles can be present on the screen and need not further disintegrate, although typically such particles are too small to be held by the screen mesh and thus are also not present as a residue on the screen. According to the invention, the tablets of the present invention disintegrate in less than 60 seconds.

In a further aspect, the present invention provides a process for preparing an orally disintegrating pharmaceutical tablet comprising Nefopam according to the first aspect, the process comprising the steps of:
(a) Preparing pellets or granules of Nefopam;
(b) Taste masking the pellets or granules;
(c) Preparing a blend comprising various pharmaceutically acceptable inert excipients;
(d) Mixing the blend with Nefopam pellets or granules and lubricating it;
(e) Compressing the lubricated blend to form a tablet.

Mixing of the excipients can be performed in a conventional device used for mixing of powders, such as for example motionless (passive) mixers, fluidized bed, diffusion, bi-conicdiffusion, uniconic, biconic, turbular, cubic, planetary, Y-, V-shaped, and low shear or high shear mixers.

Generally the drying of the pellets or granules for example can be performed in one of the following ways: trays, fluid bed, and microwave assist/vacuum/gas stripping (one pot processing).

The description of the present invention of orally disintegrating pharmaceutical composition comprising Nefopam is further illustrated by the following nonlimiting example. However, a person skilled in the art would recognize that, the specific example is intended to illustrate, not limit, the scope of the present invention.

### EXAMPLES:

### EXAMPLE 1: Pharmaceutical composition according to the present invention

**Table 1: Pharmaceutical composition comprising Nefopam**

| **Ingredients** | **%W/W** | **Mg / tablet** |
|---|---|---|
| Non pareil seeds (250 - 355 µm) | 27.71 | 97.0 |
| Nefopam hydrochloride | 8.57 | 30.0 |
| Hypromellose | 2.29 | 8.0 |
| Isopropyl Alcohol | Q.S | Q.S |
| Purified water | Q.S | Q.S |
| Eudragit NE 30D | 8.74 | 30.6 |
| Hypromellose | 2.19 | 7.65 |
| Silica, Colloidal Anhydrous | 1.93 | 6.75 |
| Purified Water* | Q.S | Q.S |
| Mannitol | 3.71 | 13.00 |
| Cellulose Microcrystalline | 30.00 | 105.00 |
| Crospovidone | 10.00 | 35.00 |
| Sucralose | 2.00 | 7.00 |
| Silica, Colloidal Anhydrous | 1.00 | 3.50 |
| Orange flavor | 0.86 | 3.00 |
| Magnesium stearate | 1.00 | 3.50 |
| **Total** | 100.0 | 350.00 |

The manufacturing process for the preparation of a pharmaceutical composition according to example 1 comprises various steps as per paragraph [0037].

Specifically, the manufacturing process comprises the steps of,
a) Preparation of drug pellets comprising the steps of
   1. Hypromellose was added under stirring in isopropyl alcohol to form a solution;
   2. Nefopam hydrochloride was added to the solution formed in step a. to obtain a clear solution.
   3. Purified water was added to the clear solution to form a drug loading suspension.
   4. The Non pareil seeds (250 - 350 µm) were charged in fluidized air bed coater and the drug loading suspension was added to it. The pellets were formed in this step.
   5. The drug pellets were dried and sifted and milled.

The next step in the manufacturing of composition comprises the step of, b) Taste mask coating comprising the steps of
1. Hypromellose was added to purified water under continuous stirring to form a solution. Silica, colloidal anhydrous was added to the above solution to form a suspension.
2. Eudragit NE 30D was added to the suspension under stirring.
3. The pre-sifted pellets in step a) were charged in fluidized air bed coated and masked with the suspension in step 2.
4. The taste masked pellets were dried in suitable dryer.
5. The dried pellets were sifted and milled to obtain suitable pellet size.

The next step in the manufacturing of composition comprises the step of, c) preparation of a blend comprising the steps of
1. Mannitol 200 + Microcrystalline Cellulose + Sucralose + Crospovidone + Silica colloidal Anhydrous + Orange flavor were sifted, through suitable sifter to form a colour blend.
2. The pre sifted taste masked pellets were transferred to suitable blended along with the colour blend formed in step 1.
3. Magnesium stearate was sifted separately.
4. The blend formed in step b. was lubricated using sifted magnesium stearate.

The next step in the manufacturing of composition comprises the step of, d) lubrication of a blend comprising the step of
1. The blend formed in step b. was lubricated using sifted magnesium stearate.

The next step in the manufacturing of composition comprises the step of, e) tablet compression comprising the step of
1. Lubricated blend formed in step d. was compressed using suitable parameters using following parameters

| SN | Parameters | Standard |
|---|---|---|
| 1 | Appearance | White to off white round flat bevelled edge tablets with central concave depression on both the surfaces having orange odour. |
| 2 | Weight of 20 tablets | 7.00 g |
| 3 | Average weight of tablet | 350 mg |
| 4 | Thickness | 4.0 mm |
| 5 | Hardness | 35 N |
| 6 | Disintegration time | NMT 3 Minutes at 37 ± 2°C, without disc |
| 7 | Friability | NMT 1.0 % |

Nefopam Hydrochloride orally disintegrating tablets to be packed in ALU - ALU blister pack using blister packing machine.

### EXAMPLE 2: Pharmaceutical composition according to the present invention

**Table 2: Pharmaceutical composition comprising Nefopam**

| **Ingredients** | **%W/W** | **Mg / tablet** |
|---|---|---|
| Non pareil seeds (250 - 355 µm) | 27.71 | 97.0 |
| Nefopam hydrochloride | 8.57 | 30.0 |
| Hypromellose | 2.29 | 8.0 |
| Isopropyl Alcohol | Q.S | Q.S |
| Purified water | Q.S | Q.S |
| Eudragit E | 8.74 | 30.6 |
| Hypromellose | 2.19 | 7.65 |
| Silica, Colloidal Anhydrous | 1.93 | 6.75 |
| Purified Water* | Q.S | Q.S |
| Mannitol | 3.71 | 13.00 |
| Cellulose Microcrystalline | 30.00 | 105.00 |
| Crospovidone | 10.00 | 35.00 |
| Sucralose | 2.00 | 7.00 |
| Silica, Colloidal Anhydrous | 1.00 | 3.50 |
| Orange flavor | 0.86 | 3.00 |
| Magnesium stearate | 1.00 | 3.50 |
| **Total** | 100.0 | 350.00 |

The composition as described in example 2 was manufactured using the process as provided in the example 1.

## Claims

1. An orally disintegrating pharmaceutical composition in the form of a tablet comprising Nefopam and pharmaceutically acceptable inert excipients, wherein the composition comprises 5 to 10 % w/w of Nefopam as the only active ingredient with respect to the weight of the composition, and wherein the composition comprises:
(a) Nefopam in the form of taste masked pellets or granules;
(b) about 1 % to about 50 % w/w Diluent;
(c) about 5% to about 50 % w/w Disintegrant;
(d) about 0.05 % to about 2 % w/w of Glidant; and
(e) about 0.05 % to about 2 % w/w of Lubricant,
wherein the % are with respect to the total weight of the tablet, and
wherein the disintegration time of the tablet is less than 60 seconds as measured by the in vitro disintegration test described in US Pharmacopoeia 701, without disks.

2. The pharmaceutical composition according to claim 1, wherein the pellets or granules contain non pareil seeds.

3. The pharmaceutical composition according to claim 1, wherein the taste masked pellets or granules comprise aqueous dispersion of poly (meth) acrylate polymer.

4. The pharmaceutical composition according to claim 1, wherein the diluent is sugar, dextrates, dextrin, dextrose, fructose, lactitol, mannitol, sucrose, starch, lactose, xylitol, sorbitol, talc, microcrystalline cellulose, calcium carbonate, calcium phosphate dibasic or tribasic or calcium sulphate.

5. The pharmaceutical composition according to claim 1, wherein the disintegrant is starch, croscarmellose sodium, polyvinyl pyrrolidone, sodium starch glycolate or microcrystalline cellulose

6. The pharmaceutical composition according to claim 1, wherein the taste masking of the pellets or granules is done using aqueous dispersion of poly (meth) acrylate polymer.

7. A process for preparing an orally disintegrating pharmaceutical tablet comprising Nefopam according to one of the preceding claims, the process comprising the steps of:
(a) Preparing pellets or granules of Nefopam;
(b) Taste masking the pellets or granules;
(c) Preparing a blend comprising various pharmaceutically acceptable inert excipients;
(d) Mixing the blend with Nefopam pellets or granules and lubricating it;
(e) Compressing the lubricated blend to form a tablet.

## Patentansprüche

1. Oral zerfallende pharmazeutische Zusammensetzung in Form einer Tablette, umfassend Nefopam und pharmazeutisch zulässige inerte Hilfsstoffe, wobei die Zusammensetzung 5 bis 10 Gew.-% Nefopam als einzigen Wirkstoff, bezogen auf das Gewicht der Zusammensetzung, umfasst und wobei die Zusammensetzung umfasst:
(a) Nefopam in Form von geschmacksmaskierten Pellets oder Granalien;
(b) etwa 1 Gew.-% bis etwa 50 Gew.-% Verdünnungsmittel;
(c) etwa 5 Gew.-% bis etwa 50 Gew.-% Sprengmittel;
(d) etwa 0,05 Gew.-% bis etwa 2 Gew.-% Gleitmittel; und
(e) etwa 0,05 Gew.-% bis etwa 2 Gew.-% Schmiermittel,
wobei sich die %-Angaben auf das Gesamtgewicht der Tablette beziehen, und
wobei die Zerfallszeit der Tablette weniger als 60 Sekunden beträgt, gemessen durch den in der US Pharmacopoeia 701 beschriebenen In-vitro-Zerfallstest, ohne Scheiben.

2. Pharmazeutische Zusammensetzung nach Anspruch 1, wobei die Pellets oder Granulate non-pareile Samen enthalten.

3. Pharmazeutische Zusammensetzung nach Anspruch 1, wobei die geschmacksmaskierten Pellets oder Granulate eine wässrige Dispersion eines Poly(meth)acrylatpolymers umfassen.

4. Pharmazeutische Zusammensetzung nach Anspruch 1, wobei das Verdünnungsmittel Zucker, Dextrate, Dextrin, Dextrose, Fructose, Lactitol, Mannitol, Saccharose, Stärke, Lactose, Xylitol, Sorbitol, Talk, mikrokristalline Cellulose, Calciumcarbonat, dibasisches oder tribasisches Calciumphosphat oder Calciumsulfat ist.

5. Pharmazeutische Zusammensetzung nach Anspruch 1, wobei das Sprengmittel Stärke, Croscarmellose-Natrium, Polyvinylpyrrolidon, Natriumstärkeglykolat oder mikrokristalline Cellulose ist.

6. Pharmazeutische Zusammensetzung nach Anspruch 1, wobei die Geschmacksmaskierung der Pellets oder Granulate unter Verwendung einer wässrigen Dispersion eines Poly(meth)acrylatpolymers erfolgt.

7. Verfahren zur Herstellung einer oral zerfallenden pharmazeutischen Tablette, die Nefopam nach einem der vorhergehenden Ansprüche enthält, wobei das Verfahren die folgenden Schritte umfasst:
(a) Herstellung von Pellets oder Granalien von Nefopam;
(b) Geschmacksmaskierung der Pellets oder Granalien;
(c) Herstellung einer Mischung, die verschiedene pharmazeutisch zulässige, inerte Hilfsstoffe enthält;
(d) Mischen der Mischung mit Nefopam-Pellets oder -Granalien und Schmieren der Mischung;
(e) Komprimieren der geschmierten Mischung zur Ausbildung einer Tablette.

## Revendications

1. Composition pharmaceutique à désintégration orale sous forme de comprimé comprenant du Néfopam et des excipients inertes pharmaceutiquement acceptables, où la composition comprend 5 à 10 % p/p de Néfopam comme seul ingrédient actif par rapport au poids de la composition, et où la composition comprend:
(a) Néfopam sous forme de pastilles ou de granulés à goût masqué;
(b) d'environ 1 % à environ 50 % p/p de diluant;
(c) d'environ 5 % à environ 50 % p/p de désintégrant;
(d) d'environ 0,05 % à environ 2 % p/p d'additif de glissement; et
(e) d'environ 0,05 % à environ 2 % p/p de lubrifiant,
où les % se rapportent au poids total du comprimé, et
où le temps de désintégration du comprimé est inférieur à 60 secondes, tel que mesuré par le test de désintégration in vitro décrit dans US Pharmacopoeia 701, sans disques.

2. Composition pharmaceutique selon la revendication 1, où les pastilles ou les granulés contiennent des graines nonpareilles.

3. Composition pharmaceutique selon la revendication 1, où les pastilles ou granulés à goût masqué comprennent une dispersion aqueuse de polymère de poly (méth) acrylate.

4. Composition pharmaceutique selon la revendication 1, où le diluant est du sucre, du dextrate, de la dextrine, du dextrose, du fructose, du lactitol, du mannitol, du saccharose, de l'amidon, du lactose, du xylitol, du sorbitol, du talc, de la cellulose microcristalline, du carbonate de calcium, du phosphate de calcium dibasique ou tribasique ou du sulfate de calcium.

5. Composition pharmaceutique selon la revendication 1, où le désintégrant est de l'amidon, de la croscarmellose sodique, de la polyvinylpyrrolidone, du glycolate d'amidon sodique ou de la cellulose microcristalline.

6. Composition pharmaceutique selon la revendication 1, où le masquage du goût des pastilles ou des granulés est réalisé à l'aide d'une dispersion aqueuse de polymère poly (méth) acrylate.

7. Procédé de préparation d'un comprimé pharmaceutique à désintégration orale comprenant du Néfopam selon l'une des revendications précédentes, le procédé comprenant les étapes suivantes:
(a) Préparation de pastilles ou de granulés de Néfopam;
(b) Masquage du goût des pastilles ou des granulés;
(c) Préparation d'un mélange comprenant divers excipients inertes pharmaceutiquement acceptables;
(d) Mélanger le mélange avec des pastilles ou des granulés de Nefopam et le lubrifier;
(e) Compresser le mélange lubrifié pour former un comprimé.
